# EUROPEAN PATENT APPLICATION

(11) **EP 4 295 827 A1**
(43) Date of publication of application: **27.12.2023**
(21) Application number: 23180414.7
(22) Date of filing: 20.06.2023
(51) Int. Cl.: A61F 13/551, A61F 13/64

(54) **ABSORBENT ARTICLE AND RESPECTIVE DIAPER**

(30) Priority: 20.06.2022 IT 202200013027
(71) Applicant: Fippi SpA, 20123 Milano (MI) (IT)
(72) Inventor: GUARNERIO, Lorenzo, 20123 Milano (MI) (IT); GUARNERIO, Claudio, 20123 Milano (MI) (IT); TANASE, Elena, 20123 Milano (MI) (IT)
(74) Representative: Ercolani, Simone Pietro

(57) **Abstract**

Absorbent article (2) for reusable belt (1) comprising a permeable upper web (50), a waterproof lower web (51), an absorbent core (52) comprising absorbent material (52a) arranged between an upper layer (53) and a lower layer (54), said absorbent core (52) being arranged between said upper web (50) and said lower web (51), wherein the outer surface (51a) of the lower web (51) comprises at least one back area and at least one front area intended to reversibly engage with a reusable belt (1), at least part of said front and back areas being intended to overlap with said reusable belt (1), characterised in that said absorbent article (2) comprises means (58) for closing said absorbent article on itself, at least when said absorbent article is separated from said reusable belt (1), said closing means being arranged on the outer surface (51a) of said waterproof lower web (51).

## Description

### FIELD OF THE INVENTION

The present invention concerns an absorbent article and respective diaper.

In particular, this diaper consists of a reusable belt and a replaceable absorbent article that is combined with the belt. At the end of the use of the absorbent article, the latter is separated from the belt and then disposed of according to law.

### KNOWN PRIOR ART

Reusable belts are known to hold absorbent articles in position when in use.

For example, the patent EP0409307A2 in the name of Procter & Gamble Company describes a garment for collecting body dejections consisting of a belt and a disposable assembly that is removably attached to the belt. The belt can be attached and detached from this disposable assembly by means of a removable attachment of the hook and loop type (i.e., by means of a Velcro^{®} system). The belt is shaped so that it can be reused.

EP 0605014 A1 in the name of KIMBERLY-CLARK describes a disposable absorbent garment that provides for pleated straps to enable the garment to be worn. The straps can be removably constrained at the ends of the absorbent garment by means of buttons or Velcro^{®}-type material.

The pleating of the straps, which can be adjusted, has the purpose of fitting the straps to the user of the absorbent garment.

GB 2242612 A in the name of KIMBERLY CLARK CO describes a disposable diaper equipped with a stretch belt that can be removably attached to the diaper.

In particular, the belt may be inserted into slots arranged at an end of the diaper and may be equipped with a Velcro^{®}-type material at the ends of the belt to allow the closure of the same belt at the waist of the diaper user.

JP2011172793A in the name of LIVEDO CORPORATION describes a belt element for a diaper that is washable and reusable. This belt element can be removably attached to a disposable diaper through a Velcro^{®}-type material.

US 4,964,860 in the name of THE PROCTER & GAMBLE COMPANY describes an absorbent garment that consists of an absorbent portion, of the disposable type, and a reusable belt, which can be constrained to each other by means of a hook and loop attachment.

WO 2009/043101 A1 in the name of KUVER DESIGNS PTY LTD relates to a diaper comprising a body portion of the disposable type, and a detachable and reusable belt. A Velcro^{®}-type material is used to couple the belt to the body portion.

WO 2013/012374 A1 in the name of SCA HYGIENE PROD AB relates to a package comprising a certain number of diapers and a reusable belt that is removably attached to each diaper by means of Velcro^{®}. The belt can act as a gripping device for at least one diaper arranged within the aforesaid package.

While such solutions are useful to allow at least the reuse of part of an absorbent article and thus to limit the causes of pollution resulting from the use of such products, they do not always prove to be comfortable for the user. In fact, when the absorbent article should be separated from the belt and then disposed of, it has to be closed by the user in order to contain the user's dejections therein.

In light of the above, object of the present invention is to make an absorbent article and respective diaper which is more easily usable by the user.

Finally, object of the present invention is to make an absorbent article and respective diaper that is also easy to make from absorbent articles of known art.

### SUMMARY OF THE INVENTION

These and other objects are achieved by means of an absorbent article for reusable belt according to claim 1.

It should be noted that the absorbent articles for reusable belt, unlike conventional diapers, are devoid of means for holding these articles at the user's pelvis or waist, which consist of, e.g., fins or tabs equipped with reversible coupling elements, such as Velcro^{®} or detachable adhesives which, connected to each other, allow to laterally join the back portion of the absorbent article to the respective front portion, so as to form a belt area that holds the absorbent article at the user's pelvis or waist. According to the invention, this absorbent article for reusable belt comprises a permeable upper web, a waterproof lower web, an absorbent core comprising absorbent material arranged between an upper layer and a lower layer, said absorbent core being arranged between said upper web and said lower web, wherein the outer surface of the lower web comprises at least one back area and at least one front area intended to reversibly engage with a reusable belt, at least part of said front and back areas being intended to overlap with said reusable belt, characterised in that said absorbent article comprises means for closing said absorbent article on itself, at least when said absorbent article - after its use - is separated from the reusable belt, said closing means being arranged on the outer surface of said waterproof lower web. The solution allows to solve the problems of known art, related to diapers consisting of reusable belt and absorbent article. Thanks, in fact, to these closing means, it is always possible for the user to fold the diaper to contain the dejections therein (as is known to be done with the diapers of known art) and then to keep the absorbent article held in that folded position. In practice, the user will use the absorbent article and will be able to dispose of it without the fear that the absorbent article may reopen and cause the dejections to spill out.

According to a preferred aspect, the closing means are arranged on the outer surface of the waterproof lower web in an area intended not to overlap with said reusable belt. In particular, the closing means are selected from at least one button, at least one Velcro^{®} band, male/female elements or at least one adhesive band.

Furthermore, these closing means are preferably shaped to have a region fastened to the outer surface of the waterproof lower web, preferably in the back of the absorbent article, i.e. the part intended to cover the part of user's buttocks, and an adhesive region, or equipped with Velcro^{®} or clips (buttons) or otherwise, intended to engage with another part of the outer surface of the lower web, whether or not equipped with complementary closing means, such as e.g. a button, buttonhole or clip, or even with a part of the outer surface of the upper web.

Preferably, the adhesive or Velcro^{®} bands may be of the extensible type or may be equipped with a region fastened to the outer surface of the lower web and with a free adhesive region, i.e. not fastened to the outer surface of the lower web, in such a way as to allow it to hold on to the absorbent article, when rolled on itself for the disposal. Advantageously, in the event that the closing means consist of at least one adhesive band, said at least one adhesive band is covered, at least partially, by at least one removable protection strip.

In addition, said absorbent core has a first and a second longitudinal end and a first and a second transverse end, wherein said absorbent core has a first portion and a second portion on both sides of a transverse crotch line passing through said absorbent article, wherein the first transverse end is a front end intended to be positioned on the front side of a person and the second transverse end is a back end intended to be positioned on the person's back side, wherein the first portion of the absorbent core is a front portion and the second portion of the absorbent core is a back portion, said absorbent article further comprises a perimeter region which surrounds said absorbent core, wherein said upper permeable web is combined by gluing, or sealing, with said waterproof lower web, said absorbent article further comprises a further first and a further second transverse end substantially parallel to said first and said second transverse ends, wherein said further first transverse end is arranged in the proximity of said first transverse end and said further second transverse end is arranged in the proximity of said second transverse end; advantageously, said at least one adhesive band is arranged between said transverse crotch line and said further second transverse end. It should be noted that said transverse crotch line is also known by the simple expression "crotch line" and is substantially in the middle of the absorbent article, cutting it transversely.

The particular position of application of said at least one adhesive band, in particular between said transverse crotch line and said further first transverse end, is the one that has been found to be the most satisfactory in terms of practicality and speed of use in experimental trials.

Additionally, said at least one adhesive band is arranged at the second portion of the absorbent core.

Alternatively, said at least one adhesive band is arranged at said perimeter region. Furthermore, said at least one adhesive band extends parallel to said second transverse end (or to said first transverse end).

Alternatively, said at least one adhesive band extends parallel to said second longitudinal end (or to said first longitudinal end).

According to a further embodiment of the invention, said at least one adhesive band extends obliquely to said second longitudinal end.

Furthermore, said absorbent article comprises at least one attaching area in which the upper layer is combined with the lower layer, said at least one attaching area being devoid of said absorbent material, wherein said at least one attaching area comprises at least one first branch substantially arranged at said second portion of the absorbent core; said at least one first branch comprises a lower end transversal to said absorbent article, wherein said at least one adhesive band is arranged between said second transverse end of said absorbent article and a through imaginary straight line superimposed on said lower end of said at least one branch.

In practice, in case of the presence of at least one attaching area (also known as "channel" or "channel area") used according to known art for improving the outflow of dejections and increasing the exchange surface with the absorbent material that delimits such attaching area (or channel), the Applicant noted that said at least one adhesive band is preferably applied in the area of the absorbent article between said second transverse end of said absorbent article and a through imaginary straight line superimposed on said end of said at least one branch. This prevents the absorbent band from being deformed when the absorbent material contained in the absorbent core absorbs and, therefore, from losing its effectiveness in holding the absorbent article in the closed configuration.

In particular, said at least one attaching area comprises said first branch, a second branch and third branch; said first branch, said second branch and said third branch are Y-shaped, wherein said second branch and said third branch are substantially arranged at said first portion of said core, said first branch being preferably arranged along the central longitudinal axis of said absorbent article.

Finally, the invention also covers a diaper comprising an absorbent article according to one or more of claims 1 to 11 and a reusable belt comprising a central portion which can be reversibly combined with said absorbent article, a first side portion jutting from a first edge of said central portion and a second side portion jutting from a second edge of said central portion, opposite said first edge, wherein said belt further comprises means for reversibly constraining said first side portion and said second side portion to said absorbent article, at least when said first side portion and said second side portion encircle the user's waist.

At last, the solution described above allows the absorbent article to be able to be closed on itself, so as to contain the dejections therein, before being discarded by the user. In fact, the presence of the belt eliminates the need to provide, on the sides of the absorbent article, at least one of the two pairs of common adhesive tabs adapted to close the absorbent article once it has fulfilled its task. It is known, in fact, that normal diapers have four adhesive tabs arranged two by two on the longitudinal opposite sides of the diaper, at the back and the front, respectively. In the case of the solution described herein, however, the belt avoids the use of the traditional pair of tabs arranged at the back, where the belt is now positioned. This way, in the absence of the aforementioned adhesive band, the absorbent article would be difficult to close once used and, in any case, would not remain firmly closed.

### BRIEF DESCRIPTION OF THE FIGURES

These and other aspects of the present invention will be made clearer by the following detailed description of a preferred embodiment provided herein only by way of nonlimiting example, with reference to the accompanying figures, wherein:
Figure 1 is a plan view of the reusable belt of the diaper according to the invention;
Figure 2A is a plan view of the reusable belt of the diaper in accordance with a further embodiment;
Figure 2B is a plan view of the belt of figure 2A, in which the two side portions and the central one are still separated;
Figure 3 is a plan view of the diaper consisting of a reusable belt combined with a related absorbent article, according to an aspect of the invention;
Figure 4 is a cross-sectional view of the diaper and, in particular, the absorbent article, which is shown in figure 3 along the adhesive band combined with the outer surface of the same absorbent article;
Figures 5A-5E are plan views of the absorbent article in accordance with further embodiments of the invention.

### DETAILED DESCRIPTION OF A PREFERRED EMBODIMENT OF THE PRESENT INVENTION

With particular reference to these figures, 2 denotes an absorbent article for reusable belt 1 in accordance with the invention.

This absorbent article 2 for reusable belt, as known in the art and unlike conventional diapers, is devoid of means for holding it at the user's pelvis or waist, which consist of, e.g., fins or tabs equipped with reversible coupling elements, such as Velcro^{®} or detachable adhesives which, connected to each other, allow to laterally join the back portion of the absorbent article to the respective front portion, so as to form a belt area that holds the absorbent article at the user's pelvis or waist. For example, conventional diapers of known art are known to be equipped with two pairs of adhesive tabs arranged at the back and front of the absorbent article, respectively, which removably attach to each other to define a belt area that holds the diaper at the user's pelvis or waist.

Unlike this known art, the absorbent article 2 described herein is devoid of these adhesive tabs or analogue means, since it is held at the user's pelvis or waist by the reusable belt 1.

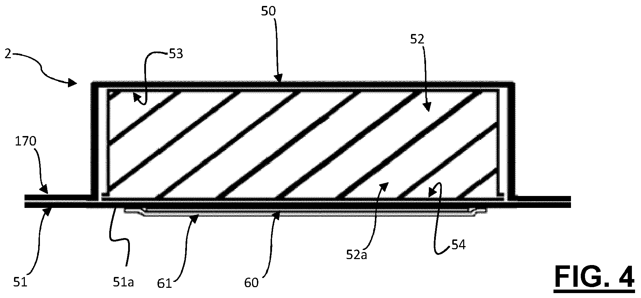

In particular, this absorbent article 2 for reusable belt 1 comprises (with reference to figure 4) a permeable upper web 50, a waterproof lower web 51 and an absorbent core 52.

The absorbent core 52, in turn, comprises an absorbent material 52a arranged between an upper layer 53 and a lower layer 54. In addition, the absorbent core 52 is arranged between the upper web 50 and the lower web 51.

The absorbent material 52a is made, in a known way, by using fluff material, typically cellulose fluff pulp. However, in other embodiments, the absorbent core may be substantially fluff-free and comprise superabsorbent polymers also known by the acronym SAP. In addition, the absorbent core 52 may comprise a combination of cellulose fluff pulp and superabsorbent polymers (SAP). These superabsorbent polymers refer to any suitable particulate (e.g., in flakes, particulate, granule or powder) or fibrous cross-linked polymer materials that can absorb at least five times and preferably at least about ten times or more its weight of a 0.9% aqueous saline solution as measured by using the Centrifuge Retention Capacity Test (EDANA 441.2-01).

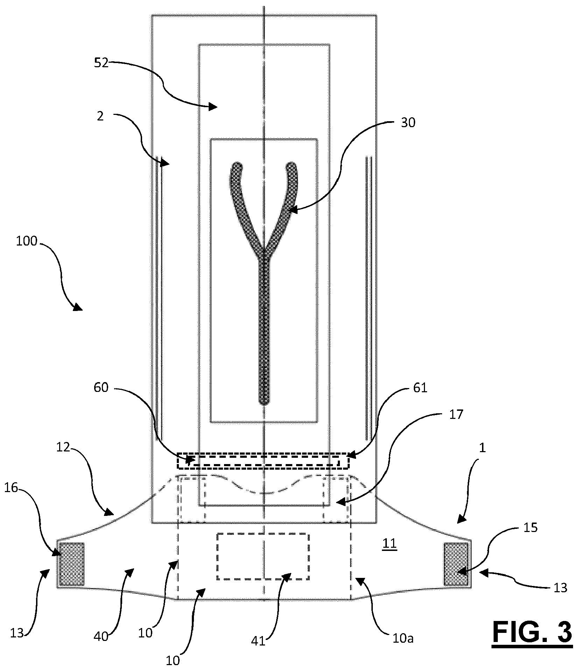

The outer surface 51a of the waterproof lower web 51, as it is understood, e.g., from figure 3, comprises at least one back area and at least one front area which are intended to reversibly, i.e. removably, engage with a reusable belt, in such a way that at least part of said front and back areas are intended to overlap with at least part of the reusable belt 1.

Advantageously, the absorbent article 2 comprises means 58 for closing said absorbent article 2 on itself, at least when the absorbent article 2 is separated from the reusable belt 1. These closing means 58 are arranged on the outer surface 51a of the waterproof lower web 51.

In particular, these closing means 58, as shown, e.g., in figure 3, are arranged in an area of the outer surface 51a of the waterproof lower web 51 that is not intended to engage, and thus overlap, with the reusable belt 1 or parts thereof.

The closing means 58 are selected from at least one button, at least one Velcro^{®} band, male/female elements or at least one adhesive band 60.

Preferably, these closing means 58 are shaped in such a way as to comprise at least one region thereof fastened to the outer surface 51a of the waterproof lower web 51, preferably in the back of the absorbent article 2, i.e. the part intended to cover the user's buttocks, and an adhesive region, or equipped with Velcro^{®} or clips (buttons) or otherwise, intended to engage with another part of the outer surface 51a of the lower web 51, which part may be equipped with or also be devoid of complementary closing means, such as e.g. a button, a buttonhole or a clip, or even with a part of the outer surface of the permeable the upper web 50.

Thus, preferably, the adhesive or Velcro^{®} bands may be of the extensible type or may be equipped with a region fastened to the outer surface 51a of the lower web 51 and with a free adhesive region, i.e. not fastened to the outer surface 51a of the lower web 51, in such a way as to allow it to hold on to the absorbent article, when rolled on itself for the disposal.

In particular, in the case of at least one adhesive band 60, it is covered, at least partially, by at least one removable protection strip 61. In the case described herein, only one adhesive band 60 is denoted, however in other alternative embodiments not shown herein, the number of adhesive bands 60 may even be greater than one, without thereby departing from the protection scope of the present invention.

According to a particular aspect of the invention, the adhesive band 60 is completely covered by the aforementioned removable protection strip 61. Once the absorbent article 2 has been used, i.e. the absorbent article 2 has been removed from the belt 1 and the protection strip 61 has been taken off, it will thus be possible to roll up the absorbent article 2 and keep it closed on itself before finally disposing of it.

This solution therefore enables the absorbent article 2 to be closed with the dejections contained therein, thus preventing it from being reopened subsequently. In fact, as will perhaps be clearer from the description below, the presence of the belt 1 eliminates the need to provide, on the sides of the absorbent article 2, the two pairs of common adhesive tabs adapted to close the absorbent article once it has been used. It is known, in fact, that normal diapers have four adhesive tabs arranged two by two on the longitudinal opposite sides of the diaper, at the back and the front, respectively. However, in the case of the solution described herein, the belt 1 makes the use of the aforementioned conventional pairs of tabs arranged at the back unnecessary. In place of these, however, the adhesive band 60 covered by the protection strip 61 is provided, which is removed when necessary, i.e. when the absorbent article 2 should be rolled up on itself at the end of its use.

It should be noted that the outer surface 5 1a of the lower web 51 refers to the surface which is opposite the surface in contact with the absorbent core 52 and which is, therefore, in contact with the external environment.

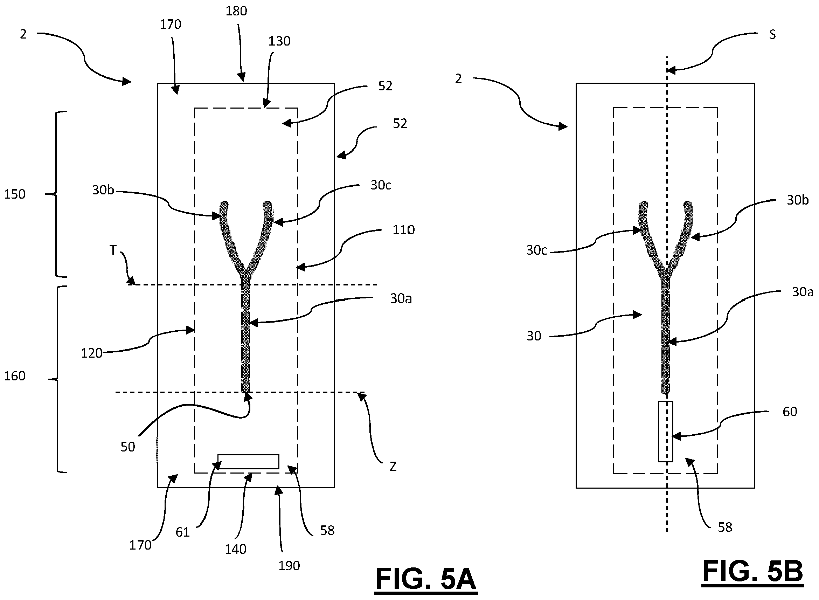

Furthermore, with reference to figures 5A-5E, the absorbent core 52 has a first and a second longitudinal end 110, 120 and a first and a second transverse end 130, 140. The absorbent core 52 has a first portion 150 and a second portion 160 on both sides of a transverse crotch line T (also known by the term "crotch line"), wherein the first transverse end 130 is a front end intended to be positioned on the front side of a person, and the second transverse end 140 is a back end intended to be positioned on the back side of the person. Therefore, the first portion 150 of the absorbent core 52 is a front portion and the second portion 160 of the absorbent core 52 is a back portion. The absorbent article 2 further comprises a perimeter region 170 which surrounds the absorbent core 52, in which the permeable upper web 50 is combined by gluing (or in another embodiment, by sealing) to the waterproof lower web 51. The absorbent article 2 further comprises a further first and a further second transverse end 180, 190, which are substantially parallel to the first and the second transverse ends 130, 140. The further first transverse end 180 is arranged in the proximity of the first transverse end 130 and the second further transverse end 190 is arranged in the proximity of the second transverse end 140. Advantageously, the adhesive band 60 is arranged between the aforementioned transverse crotch line T and the further second transverse end 190. In practice, therefore, the adhesive band 60 will always be arranged in a region of the absorbent article 2 between the transverse crotch line T and the further second end 190, thus at the area of the absorbent article 2 which will be, in use, at the back of the user.

It is noticeable from figure 3 that the adhesive band 60 is in any case arranged in an area sufficiently distant from the second transverse end 190 in such a way that between the latter and the same adhesive band 60, the overlapping and, possibly, the reversible constraint of the reusable belt 1 may take place with end areas of the outer surface 51a of the waterproof lower web 51 of the absorbent article 2.

In practice, according to an aspect of the invention, the adhesive band 60 ("disposal tape") is arranged on the outer surface 51a of the waterproof lower web 51 in such a way as not to interfere with the coupling of the same outer surface 51a of the waterproof lower web 51 with the reusable belt 1.

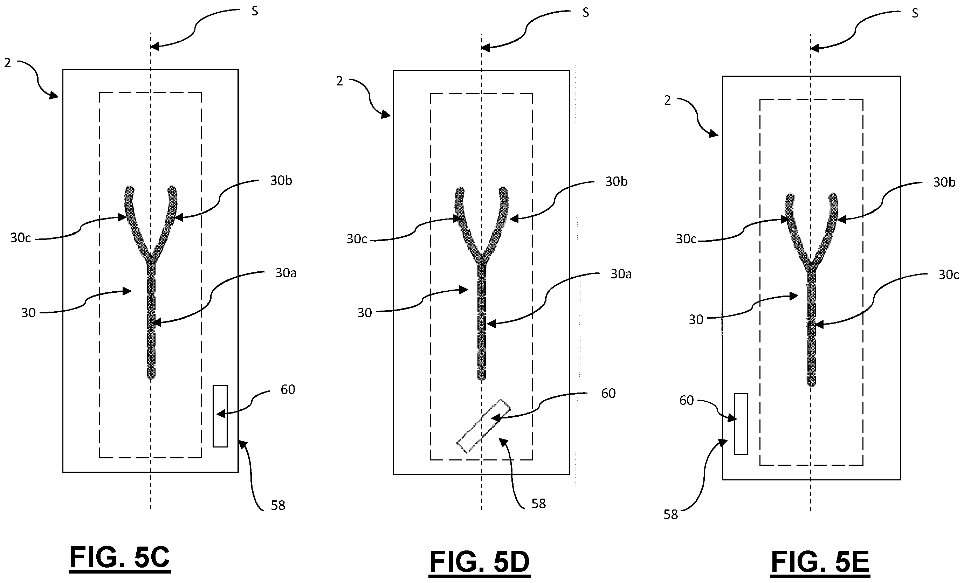

According to a particular aspect of the invention, the adhesive band 60 is arranged at the second portion 160 of the absorbent core 52 (see in particular figures 5A, 5B and 5D).

In accordance with a first variation of this embodiment, the adhesive band 60 extends parallel to the second transverse end 140 (see figure 5A).

In accordance with a second variation of this embodiment, the adhesive band 60 extends parallel to the second longitudinal end 120 (see figure 5B).

In these two cases, the adhesive band 60 may preferably be symmetrical to the longitudinal central axis S of the absorbent article. The central longitudinal axis means the axis passing through the geometric centre of the absorbent article 2, parallel to the first or the second longitudinal end 110, 120 of the absorbent core 52.

In accordance with a third variation of this embodiment, the adhesive band 60 extends obliquely to the second longitudinal end 120 of the absorbent core 52 (see figure 5D). In accordance with another embodiment, the adhesive band 60 is arranged at the perimeter region 170 of the absorbent article 2 (see figures 5C and 5E).

In the two embodiments of figures 5C and 5E, the adhesive band respectively extends parallel to and in the proximity of the second longitudinal end 120 of the absorbent core, and parallel to and in the proximity of the first longitudinal end 110 of the absorbent core.

In any case, the arrangement of the adhesive band 60 is such that it does not interfere with the reusable belt 1 when it is applied to the absorbent article 2, i.e. is such that it does not involve areas of the outer surface 51a of the lower web 51 that are intended to overlap with the reusable belt 1.

According to the particular embodiment described herein, the absorbent article 2 comprises an attaching area 30 in which the upper layer 53 of the absorbent core 52 is combined with the lower layer 54 of the absorbent core 52. This attaching area 30 (channel or channel area) is devoid of absorbent material 52a, therefore it is used for the outflow of dejections. This attaching area 30 comprises a first branch 30a substantially arranged at the second portion 160 of the absorbent core 52. This first branch 30a comprises a lower end 31 substantially transverse to the absorbent article 2. In this case, the adhesive band 60 is arranged between the second transverse end 190 of the absorbent article 2 and a through imaginary straight line Z superimposed on the lower end of the first branch 30a.

According to this embodiment, the attaching area 30 comprises, in addition to the first branch 30a, a second branch 30b and a third branch 30c. The first branch, the second branch 30b and the third branch 30c are Y-shaped. In particular, the second branch 30b and the third branch 30c are substantially arranged at the first portion 150 of the core 52. On the contrary, the first branch is arranged along the central longitudinal axis S of the absorbent article 2.

The invention also relates to a diaper 100 comprising an absorbent article 2 as described above or, in any case, according to one or more of claims 1 to 11, and a reusable belt 1 comprising a central portion 10 which can be reversibly combined with the absorbent article 2, a first side portion 11 jutting from a first edge 10 of the central portion 10 and a second side portion 12 jutting from a second edge 10b of the central portion 10, which is opposite the first edge 10a. The belt 1 further comprises means 13 for reversibly constraining the first side portion 11 and the second side portion 12 to the absorbent article 2, at least when the first side portion 11 and the second side portion 12 encircle the user's waist.

According to the embodiment described herein, the first side portion 11 and the second side portion 12 are made of an elastic material, whereas the central portion 10 is made of an inelastic material, i.e. a material with elastic capabilities close to zero. Also falling within the invention is a solution in which the central portion 10 has a reduced elastic capability compared to that of the two side portions 11 and 12.

This way, the two side portions 11 and 12 are extendable, while the central portion 10 is not. This way, it will be possible to fit the length of the two side portions 11,12 more easily to the dimensions of the user's pelvis, thus identifying the most comfortable size for the user.

It should be noted that absorbent article 2 means a product capable of absorbing the user's dejections.

Advantageously, the first side portion 11 and the second side portion 12 are made of a material distinct from the material of which the central portion 10 is made.

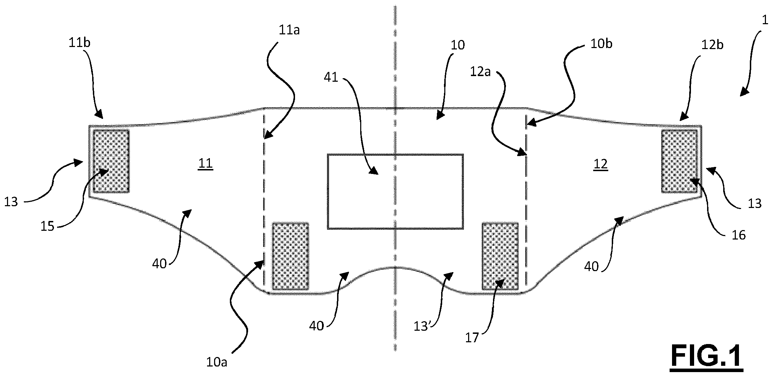

In accordance with the embodiment shown in figure 1, the material of the first side portion 11 and the second side portion 12 comprises a first fabric 40 which comprises fibres of elastic material and fibres of inelastic material, preferably the elastic material fibres comprise elastane and, even more preferably, the inelastic material fibres comprise one of polyamide, cotton, polypropylene, polyester or a combination thereof. In particular, in the specific embodiment described herein, the material of the two side portions 11 and 12 comprises a first fabric 40 which, in turn, comprises about 37% elastane fibres, 35% polyamide fibres and 28% cotton fibres for an area weight of about 375 gsm. However, in another embodiment, the percentages of elastic and inelastic fibres and the type of material used may vary without thereby departing from the protection scope of the present invention.

With this composition, the first side portion 11 and the second side portion 12 are extendable by a value higher than 15 mm.

In any case, an embodiment in which the first fabric 40 of the first side portion 11 and/or the second side portion 12 has a composition different from that mentioned above and, therefore, involves an elongation value higher than 5 mm compared to the starting value, would still fall within the scope of the protection of the present invention.

Another type of first fabric 40 could, however, be used to have an elongation of 15 mm, or another value, compared to the starting measure, without thereby departing from the protection scope of the present invention.

In addition, the central portion 10, as mentioned above, is made of an inelastic material. In particular, the material of the central portion 10 comprises the same first fabric 40 as described for the two side portions 11 and 12, which comprises fibres of elastic material and fibres of inelastic material, preferably the elastic material fibres comprise elastane and, even more preferably, the inelastic material fibres comprise one of polyamide, cotton, polypropylene and polyester or a combination thereof, and a second fabric 41 joined integrally overlapping the first fabric 40 at the central portion 10, which comprises inelastic material fibres. This second fabric 41 preferably comprises fibres chosen from polyamide, cotton, polypropylene and polyester or a combination thereof.

In the embodiment described herein, this operation of joining the second fabric 41 to the first fabric 40 is carried out by sewing and takes place by tailoring machine.

In another embodiment, this sewing operation may take place by means of a high-tech machine (e.g. for manufacturing socks) with yarns (cotton, polyamide, Lycra or other material used in these types of production). Additionally, the sewing may take place by ultrasound or by a closing system of the Velcro^{®}, buttons or glue type, without thereby departing from the protection scope of the present invention.

According to the embodiment shown in figure 1, the first fabric 40 of the first side portion 11, the second side portion 12 and the central portion 10 is made of a single piece, therefore, the two side portions 11 and 12 and the central portion 10 are joined without interruptions thanks to the first fabric 40.

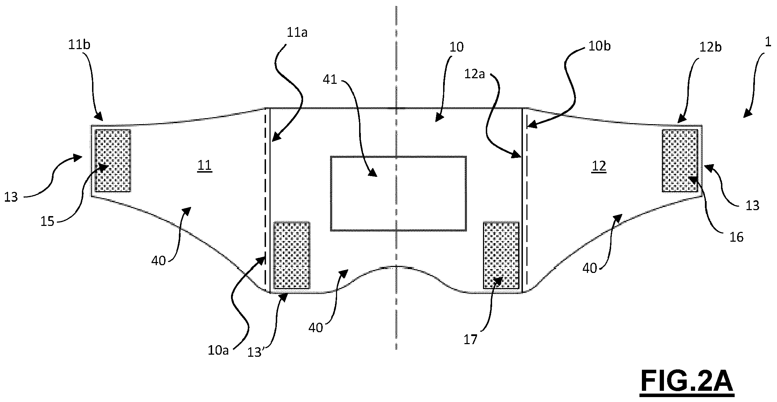

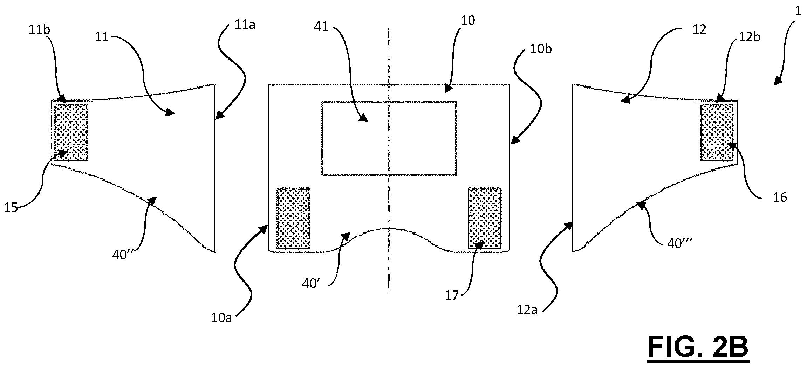

In figures 2A and 2B, however, a further embodiment of the invention is shown, wherein the first side portion 11 and the second side portion 12 are integrally constrained to the first edge 10a and the second edge 10b of the central portion 10, along the respective side connecting edge 11a, 12a present at the first side portion 11 and the second side portion 12.

According to a particular aspect of the invention, the first side portion 11 and the second side portion 12 are joined to the connecting edges 10a, 10b of the central portion 10 by sewing, along the respective side connecting edge 11a, 12a.

In the embodiment described herein, this sewing operation takes place by tailoring machine.

In another embodiment, this sewing operation may take place by means of a high-tech machine (e.g. for manufacturing socks) with yarns (cotton, polyamide, Lycra or other material used in these types of production). Additionally, the sewing may take place by ultrasound or by a closing system of the Velcro^{®}, buttons or glue type, without thereby departing from the protection scope of the present invention.

Furthermore, the means 13 for reversibly constraining the first side portion 11 and the second side portion 12 to the absorbent article 2 comprise at least one first Velcro^{®} fastening element 15 and at least one second Velcro^{®} fastening element 16, respectively. This first Velcro^{®} element 15 is combined with an end region 11b of the first side portion 11 opposite to the edge 11a and the second Velcro^{®} element 16 is combined with an end region 12b of the second side portion 12, which is opposite to the edge 12a.

In practice, the first fastening element 15 and the second fastening element 16 are combined with the absorbent article, in particular with its front portion, when the first and second side portions 11 and 12 encircle together the user's pelvis. The two fastening portions 15 and 16 are combined with the outer surface 51a of the lower web 51 of the absorbent article 2. In particular, they are combined with the first portion 150 or front portion of the absorbent article 2, which portion is intended to cover the front region of the user.

In addition, the reusable belt 1 comprises additional means 13' for reversibly combining the central portion 10 with the absorbent article 2. These additional means 13' comprise two Velcro^{®} tear-off fastening portions 17 constrained to the central portion 10 for combining the absorbent article 2 with the central portion 10 of the belt 1.

These tear-off fastening portions 17 are of the, e.g., Velcro^{®} type for combining the absorbent article 2 with the central portion 10.

These two Velcro^{®} fastening portions 17 are arranged on the two sides of the central portion 10 in the proximity of the first edge 10a of the central portion 10 and the second edge 10b of the central portion 10, respectively.

In practice, these further removable fastening means 13', constrained to the central portion 10, are combined with the absorbent article 2, in particular with its second portion 160 or back portion, when the absorbent article is arranged on the belt 1. The two fastening portions 17 are combined with the outer surface 51a of the lower web 51 of the absorbent article 2. In particular, they are combined with the back portion 160 of the absorbent article 2, which is intended to cover the back region of the user. The waterproof outer surface 51a of the upper web 51 is made of a material such that it is possible to fasten the absorbent article 2 to those removable fastening means 13 and/or to those additional removable fastening means 13'.

Figure 3 shows a diaper 100 according to the invention.

This diaper 100 comprises an absorbent article 2 and the reusable belt 1 as described above, in particular as shown in figure 1. The reusable belt 1 can be reversibly combined with the absorbent article 2 by means of additional fastening means 13' which, in this case, comprise two Velcro^{®} fastening portions 17 for combining the absorbent article 2 with the central portion 10. The number of these fastening portions 17 may vary without thereby departing from the protection scope of the present invention.

Below, the method for making the reusable belt 1 described above is also described in accordance with the two embodiments described above.

This making method comprises the step a) of conforming to size the central portion 10, the first side portion 11 and the second side portion 12, wherein the central portion 10 is made of a material having elastic capabilities lower than those of the material of which the first side portion 11 and the second side portion 12 are made. In particular, the material of the two side portions 11 and 12 is elastic, whereas the material of the central portion 10 is inelastic.

In accordance with figure 1, step a) comprises step a1) of starting from a single piece of a first fabric 40 and step a2) of cutting this single piece of the first fabric 40 to size to obtain the central portion 10, the first side portion 11 and the second side portion 12. This way, the central portion 10, the first side portion 11 and the second side portion 12 are joined in one piece without interruptions. In addition, the method comprises the step b) of integrally combining the second fabric 41 with the first fabric 40, at the central portion 10. This allows to make the central portion 10 inelastic or with little elastic capabilities compared to the two side portions 11 and 12.

Alternatively, as shown in figure 2, the step a) comprises the step a1') of starting from three distinct pieces 40',40",40''' of a first fabric 40 and the step a2') of cutting to size each piece 40',40",40" of the three pieces of first fabric 40 to obtain the central portion 10, the first side portion 11 and the second side portion 12. According to this embodiment, therefore, the central portion 10, the first side portion 11 and the second side portion 12 are physically separated from each other.

In addition, the method also comprises the step b) of integrally combining the second fabric 41 with the first fabric 40 at the central portion 10, and the step d) of integrally constraining the first side portion 11 and the second side portion 12 to the first edge 10a and the second edge 10b of the central portion 10, along the respective side connecting edge 11a,12a. Thanks to step b), the central portion 10 is made inelastic or, in any case, with little elastic capabilities compared to those of the two side portions 11 and 12.

Finally, the step b) and the step d) comprise the step of sewing the second fabric 41 to the first fabric 40 and/or the first side portion 11 and/or the second side portion 12 to the central portion 10.

This sewing step may take place by means of tailoring machine.

In another embodiment, this sewing step may take place by means of a high-tech machine (e.g. for manufacturing socks) with yarns (cotton, polyamide, Lycra or other material used in these types of production). Additionally, this sewing step may take place by ultrasound or by means of a closing system of the Velcro^{®}, buttons or glue type.

## Claims

1. Absorbent article (2) for reusable belt (1), comprising a permeable upper web (50), a waterproof lower web (51), an absorbent core (52) comprising absorbent material (52a) arranged between an upper layer (53) and a lower layer (54), said absorbent core (52) being arranged between said upper web (50) and said lower web (51), wherein the outer surface (51a) of the lower web (51) comprises at least one back area and at least one front area intended to reversibly engage with a reusable belt (1), at least part of said front and back areas being intended to overlap with said reusable belt (1), **characterised in that** said absorbent article (2) comprises means (58) for closing said absorbent article on itself, at least when said absorbent article is separated from said reusable belt (1), said closing means being arranged on the outer surface (51a) of said waterproof lower web (51).

2. Absorbent article according to claim 1, **characterised in that** the closing means are arranged on the outer surface (51a) of the waterproof lower web (51) in an area intended not to overlap with said reusable belt (1).

3. Absorbent article according to claim 1 or 2, **characterised in that** said closing means are selected from at least one button, at least one Velcro^{®} band, male/female elements or at least one adhesive band.

4. Absorbent article according to claim 3, **characterised in that** said at least one adhesive band (60) is covered by at least one removable protection strip (61).

5. Absorbent article according to one or more of claims 1 to 4, **characterised in that** said absorbent core (52) has a first and a second longitudinal end (110, 120) and a first and a second transverse end (130, 140), wherein said absorbent core has a first portion (150) and a second portion (160) on both sides of a transverse crotch line (T), wherein the first transverse end (130) is a front end intended to be positioned on the front side of a person, and the second transverse end (140) is a back end intended to be positioned on the person's back side, wherein the first portion (150) of the absorbent core is a front portion and the second portion (160) of the absorbent core is a back portion, said absorbent article further comprising a perimeter region (170) which surrounds said absorbent core (52), wherein said upper permeable web (50) is combined by gluing, or sealing, with said waterproof lower web (51), said absorbent article further comprising a further first and a further second transverse end (180, 190) substantially parallel to said first (130) and said second (140) transverse ends, wherein said further first transverse end (180) is arranged in the proximity of said first transverse end (130) and said further second transverse end (190) is arranged in the proximity of said second transverse end (140), wherein said at least one adhesive band (60) is arranged between said transverse crotch line (T) and said further second transverse end (190).

6. Absorbent article according to claim 5, **characterised in that** said at least one adhesive band is arranged at the second portion of the absorbent core.

7. Absorbent article according to claim 5, **characterised in that** said at least one adhesive band is arranged at said perimeter region.

8. Absorbent article according to one or more of claims 5 to 7, **characterised in that** said adhesive band extends parallel to said second transverse end.

9. Absorbent article according to one or more of claims 5 to 7, **characterised in that** said adhesive band extends parallel to said second longitudinal end.

10. Absorbent article according to one or more of claims 5 to 7, **characterised in that** said adhesive band extends obliquely to said second longitudinal end.

11. Absorbent article according to one or more of claims 5 to 10, **characterised in that** said absorbent article comprises at least one attaching area (30) in which said upper layer (53) of said absorbent core (52) is combined with said lower layer (54), said at least one attaching area (30) being devoid of said absorbent material (52a), said at least one attaching area (30) comprising at least one first branch (30a) arranged substantially at said second portion (160) of said absorbent core (52), said at least one first branch (30a) comprising a lower end (31) transversal to said absorbent article, said at least one adhesive band (60) being arranged between said second transverse end of said absorbent article (2) and a through imaginary straight line (Z) superimposed on said lower end (31) of said at least one first branch (30a).

12. Absorbent article according to claim 11, **characterised in that** said at least one attaching area (30) further comprises a second branch (30b) and a third branch (30c), said first branch (30a), said second branch (30b) and said third branch (30c) being Y-shaped, wherein said second branch (30b) and said third branch (30c) are arranged substantially at said first portion (150) of said absorbent core (52), said first branch (30a) being preferably arranged along the central longitudinal axis (S) of said absorbent article (2).

13. Diaper (100) comprising an absorbent article according to one or more of claims 1 to 11 and a reusable belt (1) comprising a central portion (10) which can be reversibly combined with said absorbent article, a first side portion (11) jutting from a first edge (10a) of said central portion (10) and a second side portion (12) jutting from a second edge (10b) of said central portion (10), opposite said first edge (10a), said belt (1) further comprising means (13) for reversibly constraining said first side portion (11) and said second side portion (12) to said absorbent article (2), at least when said first side portion (11) and said second side portion (12) encircle the user's waist.
